(19) Europäisches Patentamt
European Patent Office
Office européen des brevets

(11) Publication number: **0 183 002 B2**

(12) **NEW EUROPEAN PATENT SPECIFICATION**

(45) Date of publication of the new patent specification: **30.11.94**

(51) Int. Cl.⁵: **C07F 9/58**, C07D 295/10, A61K 31/675

(21) Application number: **85112179.8**

(22) Date of filing: **25.09.85**

The file contains technical information submitted after the application was filed and not included in this specification

(54) **Pharmaceutically active salt derivative of 3-hydroxy -5-(hydroxymethyl)-2-methylisonicotinaldehyde phosphate.**

(30) Priority: **26.10.84 IT 2334484**

(43) Date of publication of application: **04.06.86 Bulletin 86/23**

(45) Publication of the grant of the patent: **31.05.89 Bulletin 89/22**

(45) Mention of the opposition decision: **30.11.94 Bulletin 94/48**

(84) Designated Contracting States: **AT BE CH DE FR GB LI LU NL SE**

(56) References cited:
**BE-A- 759 608**
**BE-A- 863 754**
**DE-A- 2 006 432**
**FR-A- 2 134 218**

**CHEMICAL ABSTRACTS, vol. 69, no. 11, 9th September 1968, page 3933, column 1, abstract no. 42070c, Columbus, Ohio, US; M. KITAGAWA "Clinical and experimental studies on the effects of vitamin B6 on blood coagulation and fibrinolytic activities"; & BITAMIN vol. 37, no. 6, 1968, pages 550-562**

(73) Proprietor: **LABORATORIO ITALIANO BIOCHIMICO FARMACEUTICO LISAPHARMA S.P.A.**
**Via Licinio 11, 13, 15**
**I-22036 Erba (Como) (IT)**

(72) Inventor: **Zagnoli, Giorgio**
**via Rubini 7**
**Como (IT)**
Inventor: **Conte, Ubaldo**
**via Strada Persa 7/B**
**Pavia (IT)**
Inventor: **Colombo, Paolo**
**via Magenta 12**
**Pavia (IT)**

(74) Representative: **Gervasi, Gemma, Dr. et al**
**Studio Brevetti e Marchi**
**NOTARBARTOLO & GERVASI S.r.l.**
**33, Viale Bianca Maria**
**I-20122 Milano (IT)**



Rank Xerox (UK) Business Services
(3.10/3.09/3.3.3)

CHEMICAL ABSTRACTS, vol. 97, no. 19, 8th November 1982, page 60, column 2, abstract no. 156218r, Columbus, Ohio, US; N. MOORE et al. "Comparative vasodilator effects of five vasodilators : dihydroergotoxine, nicergoline, papaverine, naftidrofuryl and buflomedil on the dog femoral artery"; & J. PHARMACOL. vol. 13, no. 3, 1982, pages 423-430

European Conf. on Microreculation, Sept. 5-10, 1982

Thrombosis Research 13, 1017-1029, Subbarcao et al. Binding of Pircidoxalphosphate to human platelets; its effect on platelet function

Pyridoxal phosphate, inhibition of platelet junction - Kornecki-Feinberg pp. H54 to H60

Minerva medica 74, 1983 - Cajozzo et al (1983), pp. 1727-1731

## Description

The present invention relates to a novel pharmaceutically active salt derivative, and namely to 4 -(1 - pyrrolidinyl) - 1 - (2,4,6 - trimethyloxyphenyl) - 1 - butanone 3 - hydroxy - 5 - (hydroxymethyl) - 2 - methylisonicotinaldehyde - 5 - phosphate, having the following structural formula:

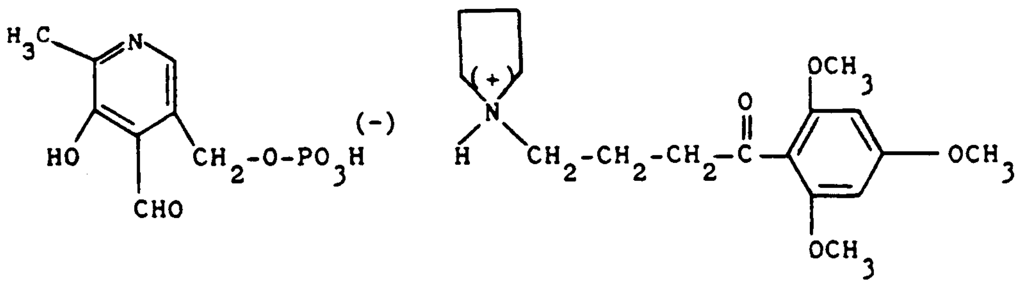

(I)

The present invention relates also to the process for the production of the salt derivative (I) and to the related pharmaceutical compositions.

The salt derivative (I) is obtained by means of the salifying of 3-hydroxy-5-(hydroxymethyl)-2-methylisonicotinaldehyde-5-phosphoric acid with 4-(1-pyrrolidinyl)-1-(2,4,6-trimethoxyphenyl)-1-butanone.

The pharmaceutical activity of the two starting products is known. In particular, the pharmacological activity is known of 4-(1-pyrrolidinyl)-1-(2,4,6-trimethoxyphenyl)-1-butanone hydrochloride (Buflomedil) on the cerebral circle for the treatment of disturbances of consciousness, and of states consequent to cerebral traumata, and its peripheral vasodilative properties are known too.

In the European Conference on microcirculation, Sept. 5-10, 1982, tests in vitro have been reported wich point out platelet antiaggregating properties of Buflomedil.

As for 3-hydroxy-5-(hydroxymethyl)-2-methylisonicotinaldehyde-5-phosphoric acid (Piridoxal phosphate), it is known as an enzyme co-factor vitamin, largely used in human therapy. BE-A-759.608 proposes the use of the salt of papaverine with pyridoxalphosphate in human therapy: this new compound affords vasodilating activity and water solubility improved in comparison with the commonly used papaverine hydrochloride.

According to Kitagawa (see C.A. *69*, 42070 c) pyridoxalphosphate per sé shows some antithrombic (anticoagulant) activity both in vitro and in vivo tests: nevertheless nothing is dislosed about the properties of pyridoxal phosphate chemically combined with other therapeutic principles. In fact the above mentioned BE-A-759.608 of a later date describes for the combination pyridoxalphosphate/papeverine merely a vasodilating activity. We have now unexpectedly found that from the reaction of the two starting products i.e. Buflomedil and pyridoxalphosphate, a drug is obtained having a remarkable antithrombic acitivity in combination with the vasodilator action of Buflomedil.

It is to be noted that none of the presently used drugs shows a combination of antithrombotic action and of peripheral vasodilative action.

The salt derivative (I) according to the present invention shows hence a platelet aggregation inhibitor and a vasodilator activity in one single molecule and is hence directed to the treatment of clinical patterns characterized by peripheral failure, clinical patterns to be found with particular frequency in old people, with a wider and more complete efficacy than Buflomedil.

The salt derivative (I) according to the invention shows moreover, relatively to Buflomedil Base, the further advantage of a high solubility in water, which favours its bioavailability and allows it to be administered by the parenteral and the rectal way.

The salt derivative (I) according to the present invention is prepared by means of a process charcterized in that 3-hydroxy-5-(hydroxymethyl)-2-methyl-isonicotinaldehyde-5-phosphoric acid is reacted with 4-(1-pyrrolidinyl)-1-(2,4,6-trimethoxyphenyl)-1-butanone in a reaction medium constituted by an organic solvent in which (I) is soluble, and the reaction product (I) is crystallized from an organic solvent wherein it is insoluble. The characteristics and the advantages of the salt derivative (I) and of the related production process shall be better evidenced by the following disclosure, which relates to a preferred embodiment.

For the preparation of the salt derivative (I), 3-hydroxy-5-(hydroxymethyl)-2-methylisonicotinaldehyde-5-phosphoric acid is previously liberated from its monosodium salt in a column with strongly basic ion exchange resin activated as chloride. The aqueous solution of 3-hydroxy-5-(hydroxymethyl)-2-methylisonicotinaldehyde-5-phosphoric acid so obtained is concentrated to dryness, a yellow oily residue being obtained, which is redispersed three times with small portions of absolute ethanol, to remove water. The residue is finally treated with anhydrous ethyl acetate, a product being obtained with is filtered and dried in a vacuum desiccator.

3-Hydroxy-5-(hydroxymethyl)-2-methylisonicotinaldehyde-5-phosphoric acid so obtained is dispersed in methanol, in which it is not soluble, and solid 4-(-pyrrolidinyl)-1-(2,4,6-trimethoxyphenyl)-1-butanone is added under stirring. The reaction mass is then heated to a temperature comprised within the range of from 40 °C to the solvent refluxing temperature, and, preferably, to a temperature comprised within the range of from 45 to 55 °C, a clear yellow solution being obtained, which even after cooling, does not yield any precipitates. The solution is concentrated up to complete removal of methanol, is then redispersed with ethyl ether, and after shaking the salt derivative (I) is obtained as crystals, which is filtered, washed with ethyl ether and dried in a vacuum desiccator.

The salt derivative (I) obtained is a crystalline powder of yellow colour, very soluble in water and aliphatic alcohols, whilst it is insoluble in ether ether. The crystallization of the raw product is carried out from a blend of aliphatic alcohols and ethyl ether, up to the required purity degree. The yield of each crystallization is of about 90%.

The characterization of said product was carried out by: X-ray diffractometry, I.R., U.V., and NMR spectrography and by means of the determination of the melting temperature and of pH.

The crystallographic analysis of X-ray diffractometry demonstrates that the product is a single substance deriving from the formation of a ionic bond between the two reactants.

In the following Table 1 the characteristic peaks (diffraction angles $\theta$ and interplanar distances d), $\lambda_{Cu}$ = 1.5418 Å, and the intensity on relative scale $I_{rel}$ are reported for the salt derivative (I) according to the invention, and, for comparison purposes, for the two reactants (Buflomedil and Pyridoxal phosphate).

| | | $2\theta_{Cu}$ | d | $I_{rel}$ |
|---|---|---|---|---|
| 1. BUFLOMEDIL Base | a | 19.7 | 4.51 | (9.0) |
| | b | 16.3 | 5.44 | (7.8) |
| | c | 15.9 | 5.57 | (7.5) |
| | d | 23.9 | 3.72 | (7.0) |
| | e | 25.7 | 3.47 | (5.8) |
| 2. SODIUM PYRIDOXAL PHOSPHATE (acid) | a | 4.2 | 21.04 | (1.6) |
| | b | 17.6 | 5.04 | (1.1) |
| 3. SALT DERIVATIVE (I) | a | 24.2 | 3.68 | (2.9) |
| | b | 20.5 | 4.33 | (2.0) |
| | c | 24.7 | 3.60 | (1.8) |
| | d | 15.3 | 5.79 | (1.6) |
| | e | 12.4 | 7.14 | (1.5) |

It results from this Table that no characteristic peaks of the reactants are present in the salt derivative (I), and vice-versa; in the salifying of the reactants a new crystalline phase has hence been obtained.

The I.R. absorption spectrum carried out in Nujol shows characteristic absorption peaks at the following wavelengths:

- at ..2720 $cm^{-1}$ absorption band of aldehydic -CH
- at ..1695 $cm^{-1}$ absorption band of ketonic carbonyl
- at ..1665 $cm^{-1}$ band of the aldehydic carbonyl
- at ..1460 and 1375 $cm^{-1}$ bands of the aromatic double bond.

The absorption spectrum of a solution in ethanol shows an absorption peak at 283 ± 1 nm.

At the NMR spectrum the following signals are detected:

| | | |
|---|---|---|
| - | at 2.1 s | 3H ($CH_3$) Pyr |
| - | at 3.7 s | 9H ($OCH_3$) B |
| - | at 3.9 s | 3H ($1CH_3$) |
| - | at 5.95 s | 2H (Aromatics) B |
| - | at 4.16 q | 1H (1CH) |
| - | at 7.22 s | 1H (Aromatics) P |
| - | at 7.95 s | 1H (Aromatics) P |
| - | at 10.4 s | 1H (aldehydic-CH) P |
| - | at 10.5 s | 1H (1NH) |

The melting temperature, as measured on Kofler block, is well defined at 77°-80°C.

pH, as determined in a 2% aqueous solution, is of 7.0. As for the pharmaceutical compositions, wherein the salt derivative (I) according to the present invention may enter as the active principle, it can be observed that, due to its high solubility in water, with it all the pharmaceutical forms can be prepared, both for oral and rectal administering, and for the injectable and topic way, in combination with the usual excipients and with the conventional pharmaceutical media.

To the purpose of illustrating, without limiting, the process according to the present invention, the following Example is reported.

Example 1

In a column about 700 ml of (moist) strongly basic ion exchange resins are activated with a 5% HCl solution, and the resin is washed up to the removal of the excess of hydrochloric acid. After that, through the column a 20% solution of 50 g of monosodium 3-hydroxy-5-(hydroxy-methyl)-2-methyl-isonicotinal-dehyde-5-phosphate is passed. The mass inside the column is finally washed with water up to neutral pH. The so-obtained aqueous solution of 3-hydroxy-5-(hydroxymethyl)-2-methyl-isonicotinaldehyde-5-phosphoric acid is concentrated to an oil which is redispersed three times in an amount of absolute ethanol which is nearly threefold relatively to the weight of the residue. After the removal of water, the residue is redispersed in 200 ml of anhydrous ethyl acetate, within which a yellow precipitate is formed under shaking, which is vacuum filtered.

The filtrate is washed with anhydrous ethyl acetate and vacuum dried.

An amount of 42.5 g of 3-hydroxy-5-(hydroxymethyl)-2-methylisonicotinaldehyde-5-phosphoric acid ($C_8H_{10}NO_6P$, m.w. = 247.156) ready for the salifying is obtained (yield 85%). In 380 ml of methanol 40 g of 3-hydroxy-5-(hydroxymethyl)-2-methylisonicotinaldehyde-5-phosphoric acid are dispersed and, always at room temperature and under stirring, 56 g are introduced of 4-(1-pyrrolidinyl)-1-(2,4,6-trimethoxyphenyl)-1-butanone ($C_{17}H_{26}NO_4$, m.w. = 307.4).

The system is heated at 50°C until a single phase is obtained. One is so sure that the salifying has taken place, on considering the insolubility of 3-hydroxy-5-(hydroxymethyl)-2-methylisonicotinaldehyde-5-phosphoric acid even at high temperature.

The solution obtained is cooled, is concentrated in vacuo until the methanol, solvent wherein the solubility of the salt derivative (I) is such as to prevent the crystallizing, has been completely removed. After that the methanol has been removed, the residue is dispersed in 480 ml of ethyl ether, and is then filtered and dried in a desiccator in vacuo at room temperature.

An amount of 76.8 g of 4-(1-pyrrolidinyl)-1-(2,4,6)-trimethoxyphenyl)-1-butanone 3-hydroxy-5-(hydroxymethyl)-2-methylisonicotinaldehyde-5-phosphate ($C_{25}H_{36}N_2O_{10}$, m.w. = 554.576) is obtained, with an active salifying yield of 80%. The salt derivative (I) obtained has a melting point of 77-80°C.

The subsequent crystallizations from methanol-ethyl ether yield the product with the desired purity level.

**Claims**
**Claims for the following Contracting States : BE, CH, DE, FR, GB, LI, LU, NL, SE**

**1.** Pharmacologically active salt derivative constituted by 4 - (1 - pyrrolidinyl) - 1 - (2,4,6 - trimethoxyphenyl) - 1 - butanone 3 - hydroxy - 5 - (hydroxymethyl) - 2 - methylisonicotinaldehydephosphate, having the following structural formula:

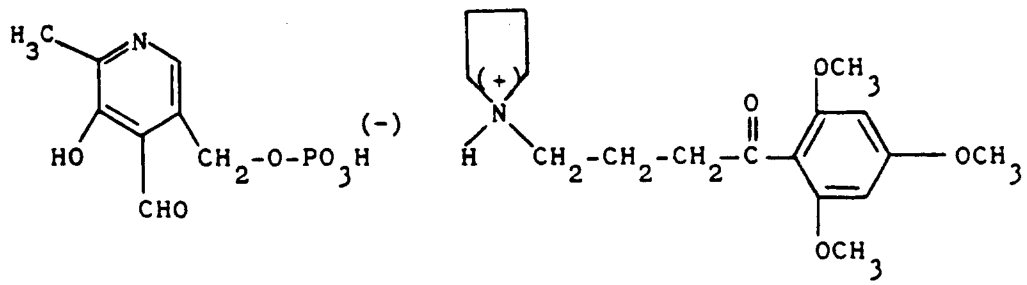

(I)

2. Process for the preparation of the pharmacologically active salt derivative constituted by 4 - (1 - pyrrolidinyl) - 1 - (2,4,6 - trimethoxyphenyl) - 1 - butanone 3 - hydroxy - 5 - (hydroxymethyl) - 2 - methylisonicotinaldehyde phosphate having the following structural formula:

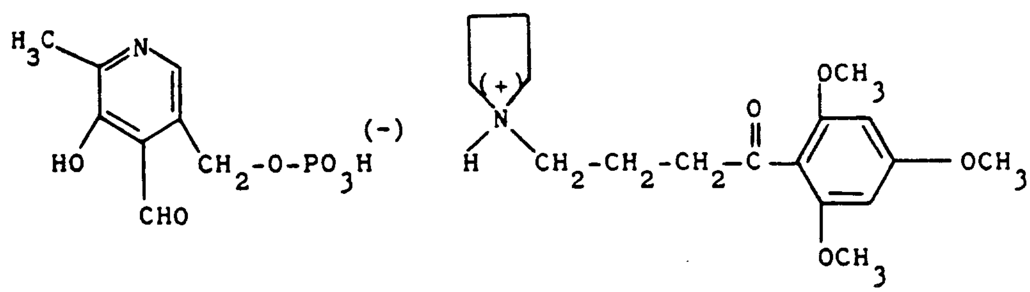

(I)

characterized in that 3-hydroxy-5-(hydroxymethyl)-2-methylisonicotinaldehyde-phosphoric acid is reacted with 4-(1-pyrrolidinyl)-1-(2,4,6-trimethoxyphenyl)-1-butanone in a reaction medium constituted by an organic solvent in which (I) is highly soluble, and the reaction product (I) is crystallized from an organic solvent in which its solubility is low.

3. Process according to claim 2, characterized in that said reaction is carried out at a temperature comprised within the range of from 40 °C to the refluxing temperature of the solvent, and preferably at a temperature comprised within the range of from 45 to 55 °C.

4. Process according to claim 2, characterized in that said reaction medium is constituted by an aliphatic alcohol containing from 1 to 4 carbon atoms.

5. Process according to claim 2, characterized in that the reactants are reacted in a nearly stoichiometric molar ratio.

6. Process according to claim 2, characterized in that said crystallization solvent is constituted by mixtures of aliphatic alcohols and ethyl ether.

7. Therapeutic compositions comprising the salt derivative (I) as the active principle, both for oral and rectal administration, and for injectable or topical administration, in combination with the usual excipients and pharmaceutical media.

**Claims for the following Contracting State : AT**

1. Process for the preparation of the pharmacologically active salt derivative constituted by 4 - (1 - pyrrolidinyl) - 1 - (2,4,6 - trimethoxyphenyl) - 1 - butanone 3 - hydroxy - 5 - (hydroxymethyl) - 2 - methylisonicotinaldehyde phosphate having the following structural formula:

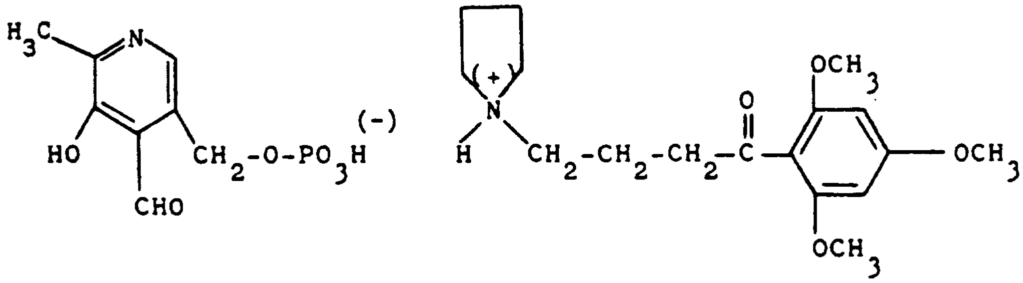

(I)

characterized in that 3-hydroxy-5-(hydroxymethyl)-2-methylisonicotinaldehyde-phosphoric acid is reacted with 4-(1-pyrrolidinyl)-1-(2,4,6-trimethoxyphenyl)-1-butanone in a reaction medium constituted by an organic solvent in which (I) is hhighly soluble, and the reaction product (I) is crystallized from an organic solvent in which its solubility is low.

2. Process according to claim 1, characterized in that said reaction is carried out at a temperature comprised within the range of from 40 ° C to the refluxing temperature of the solvent, and preferably at a temperature comprised within the range of from 45 to 55 ° C.

3. Process according to claim 1, characterized in that said reaction medium is constituted by an aliphatic alcohol containing from 1 to 4 carbon atoms.

4. Process according to claim 1, characterized in that the reactants are reacted in a nearly stoichiometric molar ratio.

5. Process according to claim 1, characterized in that said crystallization solvent is constituted by mixtures of aliphatic alcohols and ethyl ether.

**Patentansprüche**
**Patentansprüche für folgende Vertragsstaaten : BE, CH, DE, FR, GB, LI, LU, NL, SE**

1. Pharmakologisch aktives Salzderivat bestehend aus 4-(1-Pyrrolidinyl)-1-(2,4,6-trimethoxyphenyl)-1 - butanon-3-hydroxy-5-(hydroxymethyl)-2-methylisonicotinaldehyd-phosphat der folgenden Strukturformel

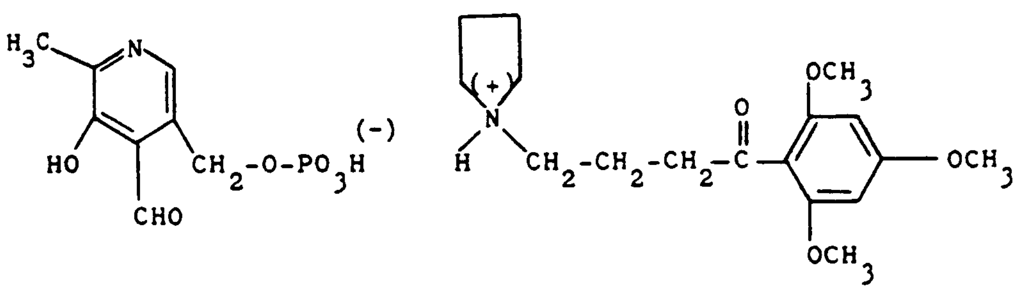

(I)

2. Verfahren zur Herstellung des pharmakologisch aktiven Salzderivates bestehend aus 4-(1-Pyrrolidinyl)-1-(2,4,6-trimethoxyphenyl)-1-butanon-3-hydroxy-5-(hydroxymethyl)-2-methylisonicotinaldehyd-phosphat der folgenden Strukturformel

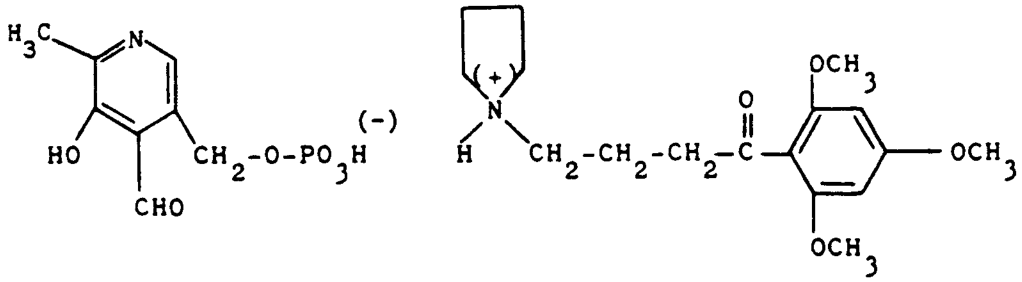

(I)

dadurch gekennzeichnet, daß 3-Hydroxy-5-(hydroxymethyl)-2-methylisonicotinaldehydphosphorsäure mit 4-(1-Pyrrolidinyl)-1-(2,4,6-trimethoxyphenyl)-1-butanon in einem Reaktionsmedium, das aus einem organischen Lösungsmittel besteht, in dem (I) hoch löslich ist, umgesetzt und das Reaktionsprodukt (I) aus einem organischen Lösungsmittel, in dem seine Löslichkeit gering ist, kristallisiert wird.

**3.** Verfahren nach Anspruch 2, dadurch gekennzeichnet, daß die Reaktion bei einer Temperatur im Bereich von 40 ° C bis zur Rückflußtemperatur des Lösungsmittels, vorzugsweise bei einer Temperatur im Bereich von 45 bis 55 ° C, durchgeführt wird.

**4.** Verfahren nach Anspruch 2, dadurch gekennzeichnet, daß das Reaktionsmedium aus einem aliphatischen Alkohol mit 1 bis 4 Kohlenstoffatomen besteht.

**5.** Verfahren nach Anspruch 2, dadurch gekennzeichnet, daß die Reaktanten in nahezu stöchiometrischem Molverhältnis umgesetzt werden.

**6.** Verfahren nach Anspruch 2, dadurch gekennzeichnet, daß das Kristallisationslösungsmittel aus Mischungen von aliphatischen Alkoholen und Äthylalkohol besteht.

**7.** Therapeutische Zusammensetzungen umfassend das Salzderivat (I) als Wirkstoff sowohl für orale als auch rektale Verabreichung und für injizierbare oder topische Verabreichung in Kombination mit den üblichen Exzipienten und pharmazeutischen Medien.

**Patentansprüche für folgenden Vertragsstaat : AT**

**1.** Verfahren zur Herstellung des pharmakologisch aktiven Salzderivates bestehend aus 4-(1-Pyrrolidinyl)-1-(2,4,6-trimethoxyphenyl)-1-butanon-3-hydroxy-5-(hydroxymethyl)-2-methylisonicotinaldehyd-phosphat der folgenden Strukturformel

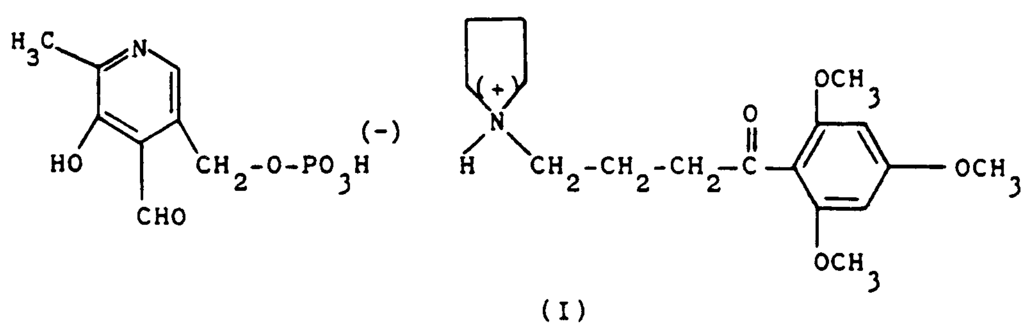

(I)

dadurch gekennzeichnet, daß 3-Hydroxy-5-(hydroxymethyl)-2-methylisonicotinaldehydphosphorsäure mit 4-(1-Pyrrolidinyl)-1-(2,4,6-trimethoxyphenyl)-1-butanon in einem Reaktionsmedium, das aus einem organischen Lösungsmittel besteht, in dem (I) hoch löslich ist, umgesetzt und das Reaktionsprodukt (I) aus einem organischen Lösungsmittel, in dem seine Löslichkeit gering ist, kristallisiert wird.

2. Verfahren nach Anspruch 1, dadurch gekennzeichnet, daß die Reaktion bei einer Temperatur im Bereich von 40 °C bis zur Rückflußtemperatur des Lösungsmittels, vorzugsweise bei einer Temperatur im Bereich von 45 bis 55 °C, durchgeführt wird.

3. Verfahren nach Anspruch 1, dadurch gekennzeichnet, daß das Reaktionsmedium aus einem aliphatischen Alkohol mit 1 bis 4 Kohlenstoffatomen besteht.

4. Verfahren nach Anspruch 1, dadurch gekennzeichnet, daß die Reaktanten in nahezu stöchiometrischem Molverhältnis umgesetzt werden.

5. Verfahren nach Anspruch 1, dadurch gekennzeichnet, daß das Kristallisationslösungsmittel aus Mischungen von aliphatischen Alkoholen und Äthylalkohol besteht.

**Revendications**

**Revendications pour les Etats contractants suivants : BE, CH, DE, FR, GB, LI, LU, NL, SE**

1. Dérivé salin pharmacologiquement actif constitué par du 3-hydroxy-5-(hydroxyméthyl)-2-méthylisonicotinaldéhyde-phosphate de 4-(1-pyrrolidinyl)-1-(2,4,6-triméthoxyphényl)-1-butanone répondant à la formule développée suivante:

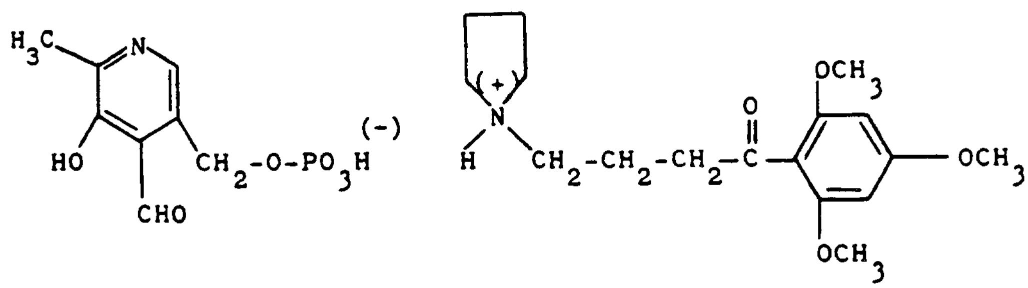

(I)

2. Procédé de préparation du sel pharmacologiquement actif constitué de 3-hydroxy-5-(hydroxyméthyl)-2-méthylisonicotinaldéhyde-phosphate de 4-(1-pyrrolidinyl)-1-(2,4,6-triméthoxyphényl)-1-butanone répondant à la formule développée suivante:

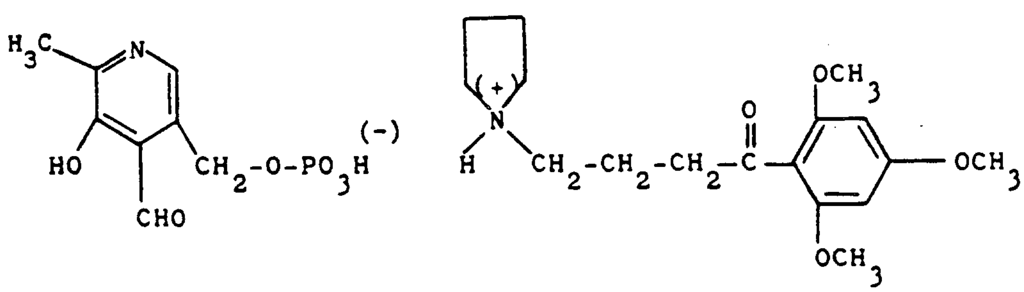

(I)

caractérisé par le fait qu'on fait réagir l'acide 3-hydroxy-5-(hydroxyméthyl)-2-méthylisonicotinaldéhyde-phosphorique avec la 4-(1-pyrrolidinyl)-1-(2,4,6-triméthoxyphényl)-1-butanone dans un milieu réactionnel constitué d'un solvant organique dans lequel (I) est trés soluble, et par le fait qu'on fait recristalliser le produit de la réaction (I) dans un solvant organique dans lequel sa solubilité est faible.

3. Procédé selon la revendication 2, caractérisé par le fait que cette réaction est effectuée à une température comprise dans l'intervalle de 40 °C à la température de reflux du solvant, et de préférence à une température comprise dans l'intervalle de 45 à 55 °C.

4. Procédé selon la revendication 2, caractérisé par le fait que ce milieu réactionnel est constitué d'un alcool aliphatique en $C_1$ à $C_4$.

5. Procédé selon la revendication 2, caractérisé par le fait que les réactifs sont mis à réagir dans un rapport molaire proche de la stoechiométrie.

6. Procédé selon la revendication 2, caractérisé par le fait que ce solvant de cristillisation est constitué de mélanges d'alcools aliphatiques et d'éther éthylique.

7. Compositions thérapeutiques comprenant le dérivé salin (I) comme principe actif, tant pour l'administration orale que pour l'administration rectale, et pour l'administration parentérale ou locale, en association avec les excipients et milieux pharmaceutiques usuels.

**Revendications pour l'Etat contractant suivant : AT**

1. Procédé de préparation du dérivé salin pharmacologiquement actif constitué par le 3-hydroxy-5-(hydroxyméthyl)-2-méthylisonicotinaldéhyde-phosphate de 4-(1-pyrrolidinyl)-1-(2,4,6-triméthoxyphényl)-1-butanone répondant à la formule développée suivante:

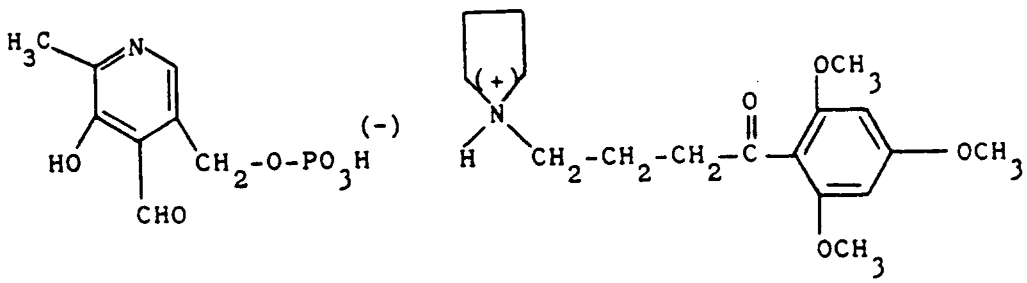

(I)

caractérisé par le fait qu'on fait réagir l'acide 3-hydroxy-5-(hydroxyméthyl)-2-méthylisonicotinaldéhyde-phosphorique avec la 4-(1-pyrrolidinyl)-1-(2,4,6-triméthoxyphényl)-1-butanone dans un milieu réactionnel constitué d'un solvant organique dans lequel (I) est très soluble, et par le fait qu'on fait recristalliser le produit de la réaction (I) dans un solvant organique dans lequel sa solubilité est faible.

2. Procédé selon la revendication 1, caractérisé par le fait que cette réaction est effectuée à une température comprise dans l'intervalle de 40 °C à la température de reflux du solvant, et de préférence à une température compise dans l'intervalle de 45 à 55 °C.

3. Procédé selon la revendication 1, caractérisé par le fait que ce milieu réactionnel est constitué d'un alcool aliphatique en $C_1$ à $C_4$.

4. Procédé selon la revendication 1, caractérisé par le fait que les réactifs sont mis à réagir dans un rapport molaire proche de la stoechiométrie.

5. Procédé selon la revendication 1, caractérisé par le fait que ce solvant de recristillisation est constitué de mélanges d'alcools aliphatiques et d'éther éthylique.